# EUROPEAN PATENT APPLICATION

(11) **EP 3 474 167 A1**
(43) Date of publication of application: **24.04.2019**
(21) Application number: 17196950.4
(22) Date of filing: 17.10.2017
(51) Int. Cl.: G06F 19/18, G06F 19/24

(54) **SYSTEM AND METHOD FOR PREDICTING GENOTYPE PERFORMANCE**

(71) Applicant: Agroscope, 1260 Nyon (CH)
(72) Inventor: Herrera, Juan, 1700 Fribourg (CH); Levy, Lilia, 4242 Laufen (CH); Holzkämper, Annelie, 8193 Eglisau (CH); Pellet, Didier, 1274 Grens (CH)
(74) Representative: Veni Swiss & European Patent Attorneys

(57) **Abstract**

An automated system is described for predicting performance of a crop genotype under particular environmental conditions. The system 1 is operable in a training phase, during which crop performance information 5' and corresponding environmental data 2' are used to train a predictor model 10 using a RR-BLUP regression algorithm 9. Predictor variables 8 for the regression algorithm are generated from the environment data 2' using a standard crop model 3. In an operation mode of the system, the trained model is used to generate predicted value(s) 7 of the performance parameters 5 of a particular genotype on the basis of environmental limiting factors 8 derived using the same crop model 3 from specified environmental conditions 2. A training method, a prediction method and a computer program product are also described.

## Description

### Field of the invention

The invention relates to the modelling of crop performance. In particular, it relates to automated systems for estimating the performance of a crop genotype.

### Background of the invention

The ability to predict performance of a crop genotype under a given set of environmental conditions is of increasing importance in modern agriculture. A farmer may wish to select a genotype which is suitable for particular environmental conditions, for example, or to assess the likely effectiveness of a particular management regime, or a seed breeder may wish to validate performance of a particular genotype over a range of environmental and management conditions.

### Prior Art

Prediction of a genotype's performance is traditionally done by growing trial areas of the genotype. Multiple genotypes may be grown in each of multiple sites, for example. A farmer may make an estimate from the trial most closely located to his or her own site, and select the most performant genotype accordingly. However, this is a source of errors because geographical distance does not necessarily mean similar growing conditions. Additionally, other farmers consider the performance of genotypes as the average over all experimental sites and years of a network, and then choose what appears, on average, as the genotype with the best performance. However, testing genotypes is a slow and expensive process and a very large number of trials would be required in order to deliver anything like the resolution required to satisfy the site and environment specifications of many farmers, particularly in countries such as Switzerland, where the elevation, rainfall, radiation, temperature and other conditions may vary greatly within very short distances.

A crop model, also referred to as a crop simulation model, conventionally comprises a set of equations or other transformation functions which describe how a yield or other performance parameter of a particular species of a crop (eg a species of a grain crop such as maize, wheat, rye etc) varies with different environmental conditions (eg light, temperature, rainfall, soil composition, tillage etc). Crop models may be mechanistic, ie relying on detailed simulations of plant growth and soil processes, or they may be empirical or statistical, based on measured performance of the species under different environmental conditions. Crop models provide coarse-grained information which can predict how the species will perform under specified environmental conditions. They have been developed as an aid to planning and managing the growth of a particular crop species. A farmer intending to plant winter wheat (triticum aestivum) may refer to a crop model for that species in order to decide a suitable planting date, to plan applications of nitrogen, to monitor the crop's development, to predict an optimum harvest date, and to estimate an expected yield, for example. A crop model for a species is typically a complex, sophisticated mathematical model which has been parameterised using many trials of the species, under many different conditions. However, the coarse-grained nature of conventional crop models means that they cannot be optimised for specific local conditions, or for predicting performance of a particular genotype of the species.

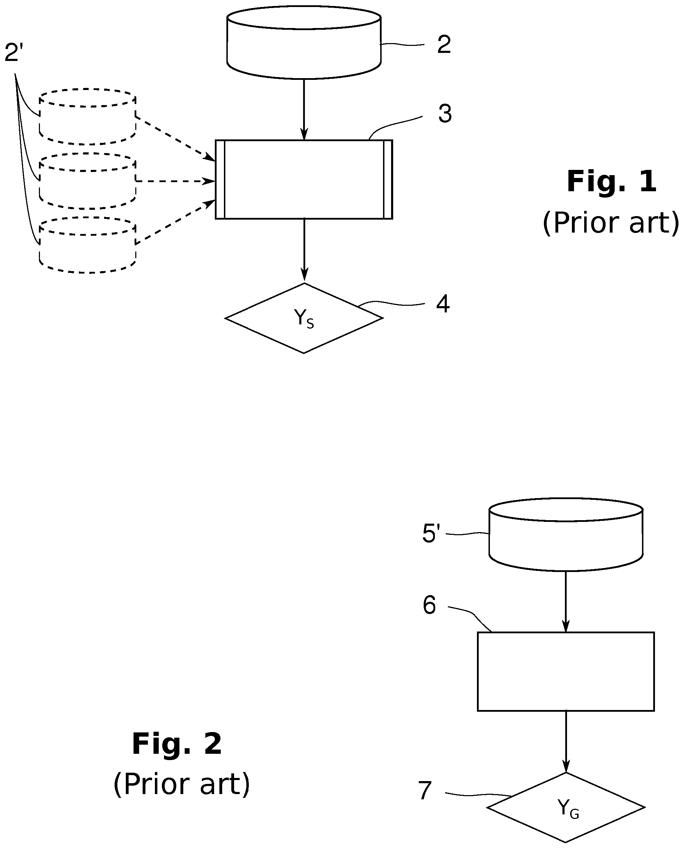

Figure 1 shows how a conventional crop simulation model 3 can be used to predict performance 4 of a given crop species under given environmental conditions 2. In the parameterisation/calibration phase, the model 3 is parameterised using data 2' from crop trials carried out under various environmental conditions. In a prediction phase, the environmental conditions 2 of a particular site are input to the trained model, which generates one or more performance parameters 4 such as the expected crop yield Ys. Crop models are widely used by agronomists, seed breeders, agricultural consultants and farmers. The crop model may be any crop model which simulates the environmental response of the species. The crop model is parameterised at the species level but not at the genotype level. A multispecies model could also be used, as long as it is suitable for characterising the growth stages and the season of the species of the subject genotype. However, single-species models are generally more accurate. Examples of commercially-available crop models include DSSAT, APSIM, Hybrid-maize, and Climate Field View.

Figure 2 shows an example of a prior art process by which a farmer could hitherto select, from performance data 5' of multiple available genotypes, a genotype suitable for a particular site or a particular type of management regime, for example. Box 6 represents a collection of possible prior art techniques for finding a genotype with optimal or adequate performance parameters such as crop yield Y_{G}. A farmer may carry out trials of multiple candidate genotypes at his/her site, for example, or s/he may research performance data of multiple genotypes grown at real sites similar to his/her site, or at scientifically-conducted genotypic trials. In reality, there is a practical limit to the amount of time a farmer can spend researching or trialling different genotypes, so in practice a farmer may try a few different genotypes and eventually settle for a particular genotype which delivers adequate but sub-optimal yield, referring to published data of average genotype performance over an experimental network, frequently with multiannual results. If the farmer is growing bread wheat for a particular mill, then the mill, or a collecting centre serving the mill, may stipulate a genotype based on its historical experience of different genotypes in different environments.

Genomic selection methods have been proposed for crop breeding programmes, by predicting genotype performance at the molecular marker level. In an article entitled "Integrating environmental covariates and crop modeling into the genomic selection framework to predict genotype performance by environment interactions" by Heslot et al, 31 October 2013, Springer Verlag, it has been proposed to use a regression analysis technique for using a crop model in combination with historical breeding data to perform genomic selection at the marker level. However, while the proposed genomic selection method may be of interest to the breeders of future genotypes, it cannot be used to predict how an available genotype will perform in unobserved conditions, and it is of no direct use to the farmer who may want to know, for example, which of a particular set of available genotypes he/she should plant in a particular field in order to maximise performance (yield).

There is therefore a need for a system which is capable of predicting the performance of a given genotype under given environmental conditions.

### Brief description of the invention

The present invention aims to overcome at least some of the above disadvantages of the prior art. To this end, an electronic system according to the invention is described in the attached claim 1, a model-training method according to the invention is described in claim 8, a prediction method according to the invention is described in claim 9 and a computer program product according to the invention is described in claim 13. Further variants of the invention are described in the dependent claims.

The inventive system and methods enable species-level environmental data to be combined with genotypic performance data of one or more genotypes by means of a first processing module implementing a crop model of the species, and a second processing module implementing a machine-learning linear regression model, so as to generate performance data for the subject genotype under the specified environmental conditions. When compared with observed input data, the inventive method may be capable of generating output data which reproduces 80% or more of the variability of the observed genotypic performance data (if such data is available). This means that the determination coefficient R2 of the regression observed versus simulated genotypic performance data (eg yield or quality parameter) is at least 0.8, and may be larger than 0.8 and smaller than or equal to 1.0.

Performance parameters typically include a measure of the yield of the crop (eg in tonnes per hectare), but may include other values such as phenological characteristics, protein content, oil content, total biomass production or nutrient uptake by the crop (N, P, K or other macro- or micro-nutrient) or soil nitrogen depletion, for example, depending on the type of crop and the purpose for which the crop is grown. Furthermore, different performance parameters may be relevant in different phenological phases of the crop.

Environmental factors may include one or more variable parameters related to climate, such as rainfall, radiation, wind or temperature. Alternatively, or in addition, they may include one or more parameters of a particular site or region, such as elevation above sea level, geology, topology, soil type, soil characteristics, slope angle and direction or drainage. Alternatively, or in addition, they may include one or more parameters relating to crop or land management, such as drilling date, tillage, amount(s) and date(s) of nitrogen application(s), irrigation patterns, or applications of pesticides, fungicides or herbicides.

The term "machine learning" may refer to a model implementing a multivariate multiple linear regression algorithm which is used for generating one or more output parameter values, for example. The model may be implemented as software or hardware. It may be implemented as a layered device such as a neural network.

The term "module" may refer to a dedicated electronic circuit or hardware device, or it may refer to a programmable processing device which has been programmed to carry out a particular function. References to a device may refer to a single physical unit or multiple interconnected units. A "system" may comprise multiple modules of the similar or different types which may be collocated or separated across a network such as the internet.

The invention will be described in detail with reference to the attached drawings, in which:
Figure 1 shows a prior art method of using a crop model to predict performance of a crop species.
Figure 2 shows a prior art method of using genotype trials data to predict genotype performance.
Figure 3 shows an example of a system according to the invention.
Figure 4 shows an example of a training part of a method according to the invention.
Figure 5 shows an example of a prediction part of a method according to the invention.

It should be noted that the figures are provided merely as an aid to understanding the principles underlying the invention, and should not be taken as limiting the scope of protection sought. Where the same reference numbers are used in different figures, these are intended to indicate similar or equivalent features. It should not be assumed, however, that the use of different reference numbers is intended to indicate any particular degree of difference between the features to which they refer.

### Detailed description of the invention

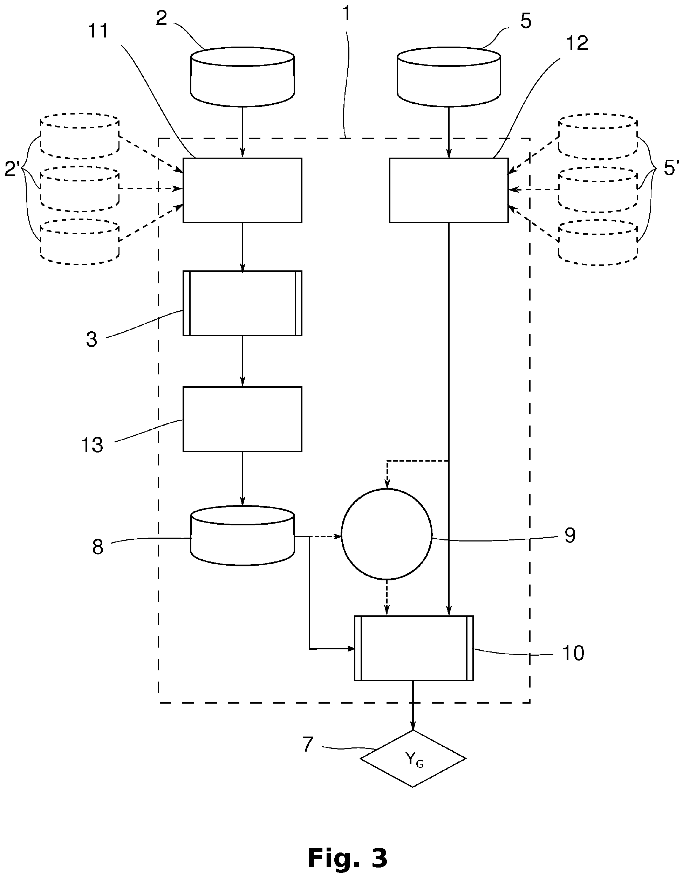

Figure 3 shows a simplified schematic view of a system 1 according to the invention. The system 1 may be partially or entirely implemented as an application program running on a computing device such as a phone or a desktop PC or laptop computer, for example. Alternatively, or in addition, the system may be partially or entirely implemented on a server, accessible via a browser or an app running on such a computing device. Where dashed paths are shown as alternative data routes to solid paths, the dashed paths indicate data routes during a training phase of the system. Input means 11 and 12 are provided for inputting environmental data 2, 2' and genotype data 5 or genotypic performance data 5' respectively. First processing module 3 is provided for implementing the functions of a crop model for the species concerned (eg bread wheat, triticum aestivum). The crop model (eg wheat suitability model) 3 is pre-trained and configured to generate values of one or more predetermined environmental limiting factors 8, ie environmental factors which limit growth in the species concerned. The predetermined environmental limiting factors (ELFs) 8 together form a feature vector for training and operating regression model 10. In the case of winter wheat, the predetermined ELFs may comprise six agro-climatic factors representing particularly significant crop limitation (stress) factors in each of four phenological phases, for example:
- Tmin (minimum temperature) - characterises frost stress
- avgT (average temperature) - effect of too cool/too warm weather
- Tmax (maximum temperature) - characterises heat stress
- RadT (average photothermal quotient) - radiation deficit
- avgWD (average water availability) - drought or water excess
- Len (length of phenological phase) - phase too long/too short

In the example of winter wheat, the four phenological phases may be:
- planting to 3-leaf stage
- 3-leaf stage to double ridge
- double ridge to anthesis
- anthesis to physiological maturity

The above environmental limiting factors (ELFs) are merely illustrative examples. In particular, ELFs are not limited to climatic factors. They may include other stress variables such as nitrogen availability, disease, management or other factors, for example. Furthermore, ELFs may be more performance-limiting in some phases than others, and the crop model may be configured such that the ELFs are weighted accordingly. Such weighting and other filtering functions may be performed by ELF vector processing module 13, which generates the ELF values for use as predictor variable (covariate) inputs to the regression model 10 and the second estimation module 9.
- Estimator module 9 is configured to, when the system 1 is in a training (parameterising) mode, implement a regression algorithm on genotypic performance data 5' using ELF feature vector values 8 as predictor input, and thereby training the predictor model implemented by second processor module 10 such that the latter can, when the system is operated in prediction mode, generate performance data 7 (eg yield Y_{G}) for the subject genotype 5. Genotypes can be defined by their name and/or a set of molecular markers of different kinds (eg AFLPs, SSRs, SNPs).
- Estimator module 9 preferably uses a best linear unbiased prediction (BLUP) regression algorithm, as this allows additional parameters to be added. More preferably, a ridge-regression BLUP (RR-BLUP) algorithm may be used for training the model 10 or any other algorithm of the "machine learning" or "deep learning" categories. Genotype trial data may be inherently multiply collinear, in particular because it may span multiple series from successive years, and RR-BLUP enables the model 10 to mitigate the difficulties associated with multiple regression and multicollinearity.

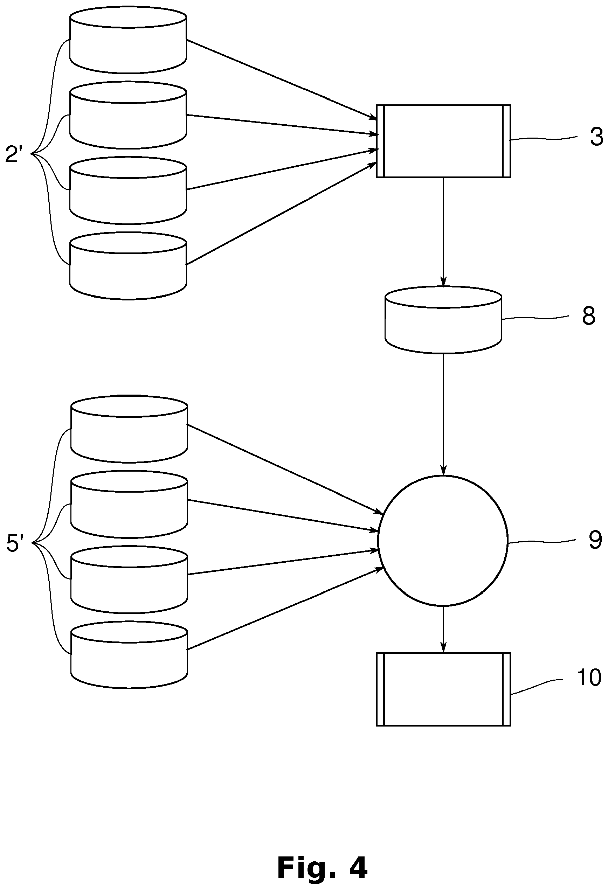

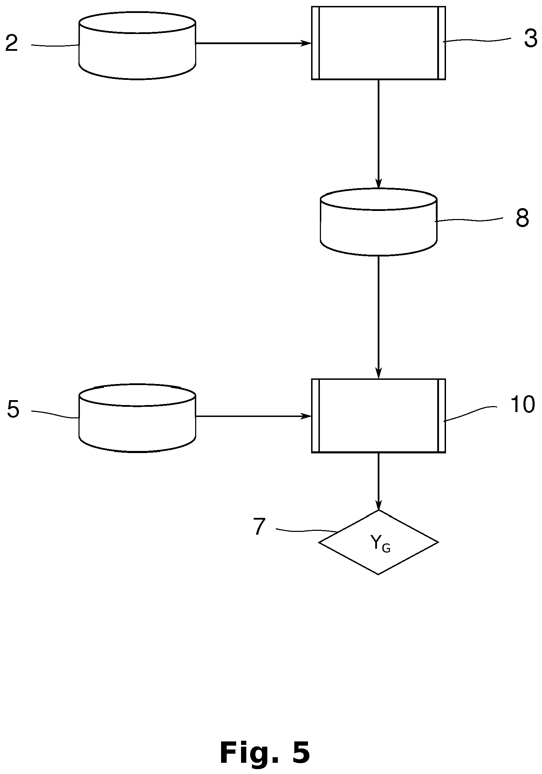

Figures 4 and 5 show an example of a method according to the invention. Figure 4 shows how the prediction model 10 may be trained with environmental limiting factors (ELF) values 8 derived by inputting environmental data 2' for the species to the crop model 3 as predictors, and genotype-specific performance data 5' as response parameters for the estimator 9 to train model 10. Figure 5 shows how the trained prediction model 10 may then be used, still with the species-specific crop model 3 for generating an ELF predictor vector from environmental data 2 of the proposed genotype cultivation, to predict performance for the particular subject genotype.

System 1 can cope with a large number of ELFs 8 and genotypes 5. In the examples developed thus far, 24 ELFs are used. The system may alternatively be used with a simple model using only one limiting factor, in which case, due to the simplicity of this example, a regular linear regression could be used. The inventive system 1 may be used simply to estimate viscosity of triticale based on average temperature cumulated over a 20 days period 24-43 days after heading (here the model 3), for example. In the training phase of system 1, temperature data 2' from genotype trials at different locations are aggregated to sums of temperature over a 20 days period 3 (24 - 43 days after heading of the genotypes) to obtain the ELF "temperature" 8. Observed viscosity (after harvest) of triticale grain of various genotypes 5' over different locations are run through the machine learning algorithm 9 to obtain for each genotype (slope and intercept of the linear regression viscosity as a function of average mean temperature). In the operating mode the system 1 uses temperature data 2 of an unobserved environment to aggregate the "sum of temperature" 8 through the crop model 3, of the sensitive 20 days period. The input variable 8 is run through the "viscosity regression" model 10 calibrated for the genotype 5. The viscosity 7 of genotype 5 is thus predicted for the particular environment condition 2.

The following further non-limiting examples are intended to aid an appreciation of the function and advantages of the inventive system and method.

In a first example scenario, a farmer wishes to select a wheat variety to grow on a particular area of land. He needs to select the genotype which will give him the highest yield, while nevertheless ensuring a minimum protein concentration (GPC) of 8%. He has the data from each of the trials, including the location (latitude and longitude) of the site and environmental data (drilling date, climatic data, management data, growth stages, yield and GPC figures). He also has access to weather data for the location and time periods of the trials, and typical/predicted weather data for his proposed growing location over the proposed growing period.

Firstly, the farmer runs the models in training mode to train 9 the machine-learning model 10 by inputting the trials performance data 5' (yield, GPC) and the environmental data 2' (weather data, drilling date, management data etc). The predetermined ELFs 8 (eg the six listed above) are generated based on the output of the crop model 3, and these are then input as predictor variables to the genotype model 10. Having trained 9 the model 10 on the trials data, the farmer then runs the system in predicting mode. His input to the model 10 is the identifier of a selected subject genotype (one of the genotypes which was featured in the trials) and one or more selected performance parameters. His input to the crop model 3 is the expected/predicted/planned environmental parameters for his proposed growing site, and the same environmental limiting factors 8 as in the training phase are generated and input as predictors to the regression model 10. The model 10 then generates predicted values of the selected performance parameters for the selected genotype, based on the environmental limiting factors 8. The prediction process can be repeated for some or all of the available trial genotypes in order to establish which is likely to provide the optimum yield under the constraint of a minimum GPC.

In a second example scenario, a farmer who has already made two applications of nitrogen to the wheat at his site would like to predict how much difference a third application would make to the final yield and protein concentration in the grain. He follows the same procedure as above. In this case, because the crop is already in its third or fourth growing phase, the farmer can include the real environmental and performance data of the crop in the training of the model 10. In the prediction mode, the farmer then runs the model multiple times in or the particular genotype, changing the environmental inputs 2 for each pass, to reflect the different situations with and without a third nitrogen application. He may also try different passes with several different timings of the third application. Each pass generates predicted performance values (yield, protein concentration etc), so the farmer can judge the likely effect of a third nitrogen application.

In a third example scenario, one of the environmental parameters 2 input to the crop model 3 is a simple binary parameter indicating whether the year will be an "el Niño" year or not, where the el Niño parameter is also included in the environmental training data for the species. A seed merchant uses the model in prediction mode, specifying the el Niño value for the coming growing season, and runs the model for each available genotype and for each region or each region type. This enables the seed merchant to advise purchasers what performance characteristics each genotype can be expected to have in different regions or different region types.

In a fourth example scenario, a grain collection centre is contracted to provide a certain quantity of wheat having a protein concentration of at least 14%. The centre manager would like to compile a map of which genotypes can be grown in which regions in order to ensure a protein concentration of at least 14%. Historical climatic data is available for the region at a resolution of 2km x 2km squares. The collection centre then runs the prediction model for each 2km x 2km square, and informs the farmer at that square which of the available genotype(s) he or she can grow in order to meet the protein concentration requirements.

In a fifth example scenario, a breeder uses the system 1 in the prediction mode to compare genotypes for tolerance to one environmental limiting factor 8. He can compare various genotypes for which system 1 has been trained and compose an artificial environment with no limiting factor but one, and have the one limiting factor he wants to score his genotypes against (for example water stress). By predicting genotypes performance in environments with this varying stress, he can compare the tolerance of his genotypes to this limiting factor and use this information to identify genotypes as parents for breeding crosses.

The system 1 shown in figure 3 may be provided with one or more automated data retrieval means configured for automatically, for example upon instruction of a user, or in dependence on a requirement of the first or second input modules 11, 12, retrieving environmental data 2, 2' and/or genotypic performance data 5, 5' from a remote eg networked device such as a server. Meteorological data providers and agricultural research establishments may provide APIs for remotely accessing their published data online, for example, and the automated data retrieval means may thereby gain automated access to performance data of a subject genotype, and/or historical or forecast climatic data, for example.

System 1 may comprise a mapping module for acquiring geographical mapping information and environmental data associated with multiple geographical locations, and for automatically running the system 1 in prediction mode so as to generate genotype performance values for the multiple geographical locations. The mapping module may advantageously comprise a graphical rendering unit for rendering the genotype performance values in the form of a geographical map.

## Claims

1. Electronic system (1) for estimating a value of a performance parameter (7), under predetermined environmental parameters (2), of a subject genotype (5) of a predetermined crop species,
the system comprising:
a first input module (11) for receiving first input data (2) of said predetermined environmental parameters,
a second input module (12) for receiving second input data (5, 5') of the subject genotype,
a first estimating module (3, 13) configured with a crop model (3) for the crop species, whereby the first estimating module (3, 13) is configured for, in response to the first input data and based on the crop model (3), generating a plurality of environmental limiting factor values (8) for the said species,
a second estimating module (9, 10) configured for using a machine-learning linear regression or deep-learning algorithm (9) to train a prediction model (10) to calculating a value (7) of the performance parameter based on the second input data (5) of the genotype and the environmental limiting factor values (8) from the species crop model (3).

2. System according to claim 1, wherein the system is operable in a training mode, in which the prediction model (10) is trainable based on the environmental limiting factor values (8) generated from training environmental data (2') and the second input training data (5'), and a prediction mode, in which the prediction model (10) is configured for predicting the performance value (7) based on environmental limiting factor values (8) generated from environmental data of the subject genotype, and on second input data (5) of the subject genotype.

3. System according to claim 1 or claim 2, wherein the regression algorithm is a BLUP algorithm.

4. System according to claim 3, wherein the regression algorithm is an RR-BLUP algorithm.

5. System according to one of the preceding claims, wherein the performance parameter comprises one of: a crop yield, a protein concentration, an oil content, a biomass production amount, a nutrient content in biomass, a nutrient accumulation amount, an oil content, a protein content or a gluten content.

6. System according to one of the preceding claims, comprising one or more automated data retrieval means for, on instruction of a user, or in dependence on a requirement of the first or second input modules (11, 12), retrieving the environmental data (2, 2') and/or genotypic performance data (5, 5') from a remote server.

7. System according to one of the preceding claims, comprising a genotype mapping module configured for repeated operation of the prediction mode for multiple genotypes and multiple geographic regions so as to generate a map of genotypic performance by geographical region.

8. Method of generating a model for predicting performance of a genotype of a crop species under predetermined environmental conditions, the method comprising:
- using a crop model (3) for the species to generate one or more environmental limiting factors (8) of the species on the basis of data (2') of the environmental conditions,
- using a linear regression algorithm to train a predictor model (10), using the environmental factors (8) as predictor variables and performance data (5') of a plurality of genotypes of the species as response variables.

9. Prediction method of operating a predictor model trained by a method according to claim 8 to predict a value of one or more performance parameters for a predetermined genotype of the crop species under predetermined environmental conditions, the prediction method comprising:
inputting data of the environmental conditions to the crop model (3) so as to generate environmental limiting predictor values for the species,
using the trained predictor model to, on the basis of the environmental predictor values, generate the value of the or each performance parameter of the genotype.

10. Method according to claim 8 or claim 9, wherein the regression algorithm is a BLUP algorithm.

11. Method according to claim 10, wherein the regression algorithm is an RR-BLUP algorithm.

12. Method according to one of claims 8 to 11, wherein the performance parameter parameters comprise at least one of: a crop yield, a protein concentration, an oil content, a biomass production amount, a nutrient content in biomass, a nutrient accumulation amount, an oil content, a protein content or a gluten content.

13. Computer program product comprising computer-executable instructions for performing a method according to one of claims 8 to 12, wherein the instructions are stored in a non-volatile storage medium.
